# EUROPÄISCHE PATENTSCHRIFT

(11) **EP 3 650 068 B1**
(45) Veröffentlichungstag und Bekanntmachung des Hinweises auf die Patenterteilung: **13.08.2025**
(21) Anmeldenummer: 19000508.2
(22) Anmeldetag: 06.11.2019
(51) Int. Cl.: A61M 16/00, A61M 16/12

(54) **BEATMUNGSGERÄT ZUR BEATMUNG MIT SAUERSTOFF**
VENTILATION APPARATUS FOR VENTILATION WITH OXYGEN
APPAREIL RESPIRATOIRE DE RESPIRATION AVEC DE L'OXYGÈNE

(30) Priorität: 07.11.2018 DE 102018008728
(43) Veröffentlichungstag der Anmeldung: 13.05.2020
(73) Patentinhaber: Löwenstein Medical Technology S.A., 2557 Luxembourg (LU)
(72) Erfinder: Marschinke, Jörg, 56581 Ehlscheid (DE)
(74) Vertreter: Löwenstein Medical IP

(56) Entgegenhaltungen:
- WO-A1-2017/079798
- DE-A1- 102017 010 115
- DE-A1- 3 604 986
- US-A1- 2008 066 752
- US-A1- 2010 224 191
- US-A1- 2014 275 901
- US-A1- 2017 095 601
- "LIFEGARD II Patient Monitor OPERATOR'S MANUAL", 1 January 2006 (2006-01-01), pages 1 - 1, XP055209485, Retrieved from the Internet <URL:http://www.infiniti.no/upload/Bruksanvisningar/CAS/UM_ENG_LifegardII.pdf> [retrieved on 20150825]

## Beschreibung

Die Erfindung betrifft ein Beatmungsgerät und ein Verfahren zur Beatmung mit Sauerstoff, das eine Steuereinrichtung und eine Atemgasquelle umfasst, zur Regelung der arteriellen Sauerstoff-Sättigung (SpO₂) durch Vorgabe einer Sauerstoffkonzentration (FiO2) im Atemgas.

Frühgeborene benötigen häufig eine exakt dosierte Sauerstoffgabe. Das Ziel dieser Therapie ist es, die kleinen Patienten mit so viel Sauerstoff wie nötig zu versorgen, dabei aber zu hohe oder schwankende Sauerstofflevel zu vermeiden, um die Sauerstoff-Toxizität und den oxidativen Stress zu minimieren.

Ein Negativbeispiel für Sauerstoffüberversorgung ist die Frühgeborenen Retinopathie.

Obwohl das optimale Niveau der arteriellen Sauerstoff-Sättigung (SpO₂) noch diskutiert wird, gibt es Hinweise darauf, dass große Schwankungen in SpO₂ unbedingt vermieden werden sollten. Dies ist im Pflegealltag einer neonatologischen Intensivstation aber oft schwer zu erreichen. Gerade deshalb ist es sinnvoll, die Verabreichung des Sauerstoffs durch ein Beatmungsgerät von der gemessenen Sauerstoff-Sättigung abhängig zu machen oder sogar das Beatmungsgerät über die SpO₂ zu regeln. Die Erfindung betrifft daher eine exakt dosierte Sauerstoffgabe bei Patienten wie beispielsweise Frühgeborenen, Kindern oder Erwachsenen.

Es können aber auch diverse Anwendungen außerhalb des Gebietes der Medizintechnik erfolgen. Beispielsweise kann bei Beatmungsgeräten für Taucher oder Feuerwehrleute das Problem auftreten.

Die DE 360 4 986 A1 offenbart eine Vorrichtung zur Überwachung der Atmung und Sauerstoffversorgung im Schlaf. Die Vorrichtung bestimmt über Sensoren physiologische Größen, die mit der Atmung oder der Sauerstoffversorgung des Patienten verbunden sind. Bei Detektion einer mangelhaften Versorgung wird ein Atem-Weckreiz generiert.

Die US 4,875,477 offenbart eine Gesichtsmaske mit integrierten Sensoren, mit deren Hilfe Vitalfunktionen bestimmt werden können. Die Sensoren sind im Bereich der Maske angeordnet und liegen dem Gesicht des Patienten an.

Die WO2017/079798A1 offenbart die Merkmale des Präambels des Anspruchs 1.

Aufgabe der vorliegenden Erfindung ist es, ein Beatmungsgerät der einleitend genannten Art derart zu verbessern, dass eine sichere, automatisierte Beatmung möglich ist und auf Fachpersonal insofern weitgehend verzichtet werden kann.

Diese Aufgabe wird erfindungsgemäß durch die Merkmale des Hauptanspruchs gelöst.

Die Erfindung betrifft ein Beatmungsgerät zur Beatmung eines Patienten mit Vorgabewerten einer Sauerstoffkonzentration (FiO2) im Atemgas, mit einer programmierbaren Steuereinrichtung, einer Atemgasquelle, einer Sauerstoffquelle, einer Gasmischeinheit, einer Anzeige (3) und Bedienelementen (2) sowie zumindest einer Schnittstelle (8,18,28) und einem Sauerstoff-Sensor zur Bestimmung einer Sauerstoffsättigung (SpO2) des Patienten, wobei die programmierbare Steuereinrichtung zum Durchführen des folgenden Verfahrens eingerichtet ist:
Vorgabe von einem Zielwert oder Grenzwerten für die SpO2 und
Vorgabe einer FiO2 und
Vorgabe einer Regelperiode (60) für die Beatmung mit der FiO2 und
Beatmung mit einer Sauerstoffkonzentration (FiO2) im Atemgas entsprechend der Vorgabe für die Zeitdauer der Regelperiode (60)
Ermittlung von aktuellen Meßwerten für SpO2 während der Zeitdauer der Regelperiode (60) Darstellung der Meßwerte für SpO2 auf der Anzeige und
Darstellung der Vorgabe für FiO2 auf der Anzeige.

Die Darstellung der Meßwerte für SpO2 und/oder die Darstellung der Vorgabe für FiO2 kann auf der Anzeige in Form von Zahlenwerten oder als Index oder als Symbol unmittelbar oder mittelbar (abrufbar) erfolgen.

Das Verfahren beinhaltet erfindungsgemäss, dass die Beatmung mit einer Sauerstoffkonzentration (FiO2) im Atemgas entsprechend der Vorgabe für die Dauer der Regelperiode (60) erfolgt und dabei Meßwerte der SpO2 während der Beatmung aufgezeichnet werden und analysiert wird, ob die Meßwerte der SpO2 dem Zielwert entsprechen und bei einer definierten Abweichung der Meßwerte der SpO2 vom Zielwert für die nachfolgende Regelperiode (60) ein neuer Vorgabewert für FiO2 angewendet wird.

Das Verfahren beinhaltet erfindungsgemäss, dass basierend auf der Abweichung der Messwerte für SpO2 von dem Zielwert eine Veränderung für den FiO2 berechnet wird und auf der Anzeige ein Symbol (52) (beispielsweise ein Pfeil) dargestellt wird, wobei das Symbol (52) dem Betrachter eine Information darüber gibt, ob der nachfolgende Vorgabewert für FiO2 - im Vergleich zu dem aktuellen FiO2-Wert - gleichbleibt oder verändert wird. ,

Das Verfahren beinhaltet auch, dass die Dauer der Regelperiode (60) entsprechend der Abweichung der Meßwerte der SpO2 vom Zielwert für die nachfolgende Regelperiode (60) angepasst wird.

Das Verfahren beinhaltet auch, dass die Regelperiode im Bereich 20 - 240 Sekunden vorgebbar ist oder angepasst wird.

Das Verfahren beinhaltet auch, dass auf der Anzeige ein Zeitgraph für die Regelperiode (60) dynamisch dargestellt wird.

Das Verfahren beinhaltet auch, dass Meßwerte für SpO2 in einer Verlaufsform chronologisch auf der Anzeige dargestellt werden.

Das Verfahren beinhaltet auch, dass Vorgabewerte für FiO2 in einer Verlaufsform chronologisch auf der Anzeige dargestellt werden.

Das Verfahren beinhaltet auch, dass Vorgabewerte für FiO2 und/oder Meßwerte für SpO2 in Form von Verlaufskurven dargestellt werden.

Das Verfahren beinhaltet auch, dass die Verlaufskurven für SpO2 und FiO2 untereinander darstellt werden.

Das Verfahren beinhaltet auch, dass am Ende der Verlaufskurve für SpO2 der aktuelle Meßwert (31) für SpO2 dargestellt wird.

Das Verfahren beinhaltet auch, dass am Ende der Verlaufskurve für FiO2 der aktuelle Vorgabewert für FiO2 dargestellt wird.

Das Verfahren beinhaltet auch, dass am Ende der Verlaufskurve der aktuelle Vorgabewert für FiO2 und/oder der aktuelle Meßwert (31) für SpO2 mit einem (Pfeil)symbol (52) hinterlegt ist, wobei die Ausrichtung des Symbols (52) dabei dynamisch anhand der vergangenen Meßwerte angepasst wird.

Das Verfahren beinhaltet auch, dass der aktuelle Vorgabewert für FiO2 auf der Anzeige gemeinsam mit einem Symbol (52) (beispielsweise einem Pfeil) dargestellt wird, wobei die Ausrichtung des Symbols (52) dabei dynamisch derart angepasst wird, dass die Ausrichtung des Symbols dem Betrachter eine Information darüber gibt, ob der nachfolgende Vorgabewert für FiO2 gleichbleibt oder verändert wird.

Das Verfahren beinhaltet auch, dass der aktuelle Messwert für SpO2 auf der Anzeige gemeinsam mit einem Symbol (52) (beispielsweise einem Pfeil) dargestellt wird, wobei die Ausrichtung des Symbols (52) dabei dynamisch derart angepasst wird, dass die Ausrichtung des Symbols dem Betrachter eine Information darüber gibt, ob der nachfolgende Vorgabewert für FiO2 gleichbleibt oder verändert wird.

Das Verfahren beinhaltet auch, dass bei einem definierten Abfall der SpO2 eine Verkürzung der aktuellen Regelperiode (60) und eine Erhöhung der FiO2 für die nachfolgende Regelperiode (60) erfolgt.

Das Verfahren beinhaltet auch, dass für SpO2 ein Zielwert visualisiert wird und Grenzwerte visualisiert werden, wobei die Grenzwerte farblich hervorgehoben oder anders visualisiert werden als die Zielwerte.

Das Verfahren beinhaltet auch, dass lediglich innerhalb vorgebbarer Zeitspannen Meßinformationen des Sensors auswertet werden.

Das Verfahren beinhaltet erfindungsgemäss, dass die Signalqualität (40) des SpO2-Signals, beispielsweise anhand der Lichtintensität, ermittelt und auf der Anzeige derart visualisiert wird, dass eine gute Signalqualität (40) visuell anders dargestellt wird, als eine schlechte Signalqualität.

Das Verfahren beinhaltet auch, dass SpO2 Messwerte, die mit einer definiert schlechten Signalqualität (40) des SpO2-Signals, ermittelt wurden, verworfen werden.

Das Verfahren beinhaltet auch, dass die Zeitdauer einer definiert schlechten Signalqualität (40) des SpO2-Signals, ermittelt wird und die Regelperiode (60) dynamisch entsprechend der Zeitdauer der definiert schlechten Signalqualität angepasst wird.

Das Verfahren beinhaltet auch, dass der aktuelle Meßwert für SpO2 (31) auf der Anzeige in einer SpO2-Grafik (30) dargestellt wird und auch vorgegebene Grenzwerte (32) visualisiert werden, wobei Meßwerte für SpO2, die in dem Ziel-Bereich liegen visuell hervorgehoben dargestellt werden.

Die Aufgabe wird erfindungsgemäß auch dadurch gelöst, dass als ein atmungsanhängiger Parameter nicht-invasiv mindestens ein Parameter des Blutes des Patienten ermittelt wird.

Um die Bedienung des Sauerstoff-Controllers für den Anwender so einfach und intuitiv wie möglich zu machen, wurden der Steueralgorithmus und die Pulsoximetriemessung in das Beatmungsgerät integriert. Die gesamte Bedienung einschließlich Visualisierung der Messdaten und Alarmeinstellungen erfolgt über die Bedienoberfläche des Beatmungsgerätes. Besonderer Wert wurde darauf gelegt, den aktuellen Patientenstatus graphisch darzustellen und diesen dem Anwender auf einen Blick zu vermitteln. Im Gegensatz zur konventionellen, manuellen Einstellung nimmt die Erfindung dem Kliniker die Routineanpassung des inspiratorischen Sauerstoffs im Atemgas ab (FiO₂), indem es kontinuierlich den Bedarf und Zustand des Patienten überwacht und die Geräteeinstellungen entsprechend anpasst. Keinesfalls soll dabei dem Anwender die Kontrolle über die Beatmung genommen werden, er kann so vielmehr von Routineaufgaben entlastet werden. Selbstverständlich besteht jederzeit die Möglichkeit, die automatische Steuerung abzuschalten, um den Sauerstoffgehalt manuell zu regeln.

Aufgrund der kontinuierlichen Überwachung des Patienten durch den Controller, die in dieser Intensität vom Pflegepersonal selbst kaum zu leisten ist, könnten große Sättigungsschwankungen reduziert und der gewünschte SpO₂-Zielbereich dauerhafter eingehalten werden.

Als eine Ausführungsvariante wird dieser Parameter durch einen auf einer Haut des Patienten positionierten Sensor ermittelt.

Dabei können als Parameter des Blutes auch Inhaltsstoffe als Blutparameter angesehen werden.

Insbesondere ist auch daran gedacht, dass als atmungsanhängiger Parameter nicht-invasiv durch mindestens einen auf einer Haut eines Patienten positionierten Sensor mindestens ein Inhaltsstoff des Blutes des Patienten gemessen wird.

Durch nicht-invasive Bestimmung der Sauerstoffsättigung unter Beatmung lassen sich schnell Trendwerte zur Analyse der Einstellungseffektivität des Beatmungsgerätes gewinnen und Nachteile der herkömmlichen Blutgasanalyse vermeiden.

Durch nicht-invasive Bestimmung der Blutwerte, vor allem des SpO2-Wertes, und Übertragung an die Steuerung des Beatmungsgerätes werden die Beatmungsparameter automatisch vom Beatmungsgerät so eingestellt, dass ein vorgegebener Ziel-SpO2 erreicht wird.

Innerhalb einer vorgebbaren Bandbreite der Minimal- und Maximalgrenzen der Beatmungseinstellungen (Sicherheitsfunktion) erhält ein Beatmungsgerät vollständige oder begrenzte Autonomie zur Erreichung der vorgegebenen Blutgaszielwerte. Die Beatmungseinstellung, unter der der Zielwert erreicht wurde, wird vom Gerät beibehalten solange der Ziel- SpO2 innerhalb eines Toleranzbereiches verbleibt.

Bei Verlassen des Ziel- SpO2 Wertes stellt das Beatmungsgerät automatisch die notwendige Einstellungsanpassung innerhalb der erlaubten Bandbreiten nach, beispielsweise durch Veränderung der FiO2.

Durch die Verwendung des erfindungsgemäßen Beatmungsgerätes ist es möglich, den Patienten in den Norm-Bereich zu ventilieren. Dieser Wert ist bei jedem Patienten anzustreben, kann aber nicht bei jedem Patienten erreicht werden. Aus diesem Grund kann der Ziel- SpO2 Wert auch vom Arzt eingestellt und nachjustiert werden.

Eine Ausführungsform des erfindungsgemäßen Gerätes verfügt damit über eine Beatmungs-Autotitration mit Vorgabe des Zielparameters SpO2.

Dies wird beispielsweise zur Einstellung eines Patienten, welcher eine Langzeitbeatmung benötigt, genutzt, um den Arzt durch die teilweise oder vollständige Übergabe des Patienten an das Beatmungsgerät zu entlasten und dadurch Kosten einzusparen. Das Gerät kann dabei mit voreingestellten Einstellungen des Arztes und nach einer gewissen Aufzeichnungsdauer und automatischen Änderung der Einstellungen durch das Beatmungsgerät gestartet, die jeweiligen Einstellungen dem Patienten mitgeben und in seiner häuslichen Umgebung verwendet werden.

Zur besseren Reaktion auf sich langsam einstellende Krankheitsveränderungen, welche eine neue Einstellung des Gerätes erfordern, wird dem Patienten nicht nur unter Aufsicht des Arztes, sondern auch während der Beatmung zu Hause, ein Messmittel zur Bestimmung seines SPO2-Wertes angelegt. Dies kann beispielsweise am Ohr, am Finger oder jedweder anderen Stelle am Körper erfolgen. Die Kommunikation zwischen Messmittel und Gerät kann über unterschiedliche Prinzipien erfolgen: Kabelverbindung, Funk, Infrarot, Bluetooth^{®}, mechanisch, elektrisch, etc.

Das Gerät zeichnet die Veränderungen auf und gibt bei kritischen Veränderungen des Lungen- und Patientenzustandes eine Warnmeldung mit dem Hinweis, den behandelnden Arzt zu kontaktieren, aus. Alternativ kann bei bestimmten Patienten auch der Arzt eine automatische Änderung der Parameter des Beatmungsgeräts zulassen. In einer weiteren Form der Erfindung kann die Meldung an den Arzt auch direkt über eine Schnittstelle (Telefonanlage, mobiles Telefon und andere Datenübertragungsmöglichkeiten, etc.) erfolgen.

Bei bestimmten Krankheitsmustern werden die voreingestellten, bei Systemstart vorhandenen Einstellungen auch beispielsweise durch fest vorgegebene, im Beatmungsgerät gespeicherte Einstellungen ersetzt. So kann der Arzt direkt am UI (UserInterface) oder der Anzeige das Krankheitsmuster wählen und das Gerät berücksichtigt wichtige für diese Erkrankung charakteristischen Einstellungen und deren Bandbreiten und stellt in diesem Fall auch die wichtigen Einstellungen bezüglich dieses Krankheitsmusters ein.

Sämtliche Monitorfunktionen, Einstellungen der SPO2-Parameter/Alarmgrenzen und die Software selbst werden im Beatmungsgerät implementiert. Die Stromversorgung und Steuerung des Systems wird beispielsweise in die Firmware des Beatmungsgerätes integriert/verknüpft.

Eine genaue Einhaltung von Vorgabewerten kann dadurch erfolgen, dass die Veränderung des Betriebsparameters des Beatmungsgerätes innerhalb eines Regelkreises durchgeführt wird, dem als Sollwerte und Istwerte Daten des Parameters des Blutes (SpO2) zugeführt werden.

Ein nochmals verbessertes Regelungskonzept kann dadurch erreicht werden, dass zur Durchführung der Regelung eine Strategieadaption in Abhängigkeit vom Meßergebnis und von der Beatmungsform durchgeführt wird. Als besonders vorteilhaft kann auch die Benennung und/oder die Zuordnung der Strategien zu bestimmten Krankheitsmustern angesehen werden. Somit stellt das Gerät abgespeicherte Strategien mit deren Einstellungen und Bandbreiten für spezielle Krankheitsbilder bereit und kann auf diese optimal reagieren. Diese speziellen Strategien sind fest durch das Gerät vorgegeben. Daneben gibt es freie Strategien, die der Arzt nach seinen Vorstellungen konfigurieren, benennen und abspeichern kann.

Eine weitere Erhöhung der Anpassungsfähigkeit kann dadurch erfolgen, dass bei der Veränderung des Betriebsparameters eine Moduswahl durchgeführt wird.

Ein bedienungsunabhängiger Betrieb wird dadurch unterstützt, dass die Veränderung des Betriebsparameters von der Steuereinrichtung autonom durchgeführt wird.

Zur Berücksichtigung von externen Steuervorgaben wird vorgeschlagen, dass von der Steuereinrichtung Bedienereingaben ausgewertet werden.

Eine genaue Einhaltung von Sollwerten wird dadurch unterstützt, dass von der Steuereinrichtung kontinuierlich Meßinformationen des Sensors ausgewertet werden.

Ein erforderlicher Auswertungsaufwand kann dadurch vermindert werden, dass die Steuereinrichtung lediglich innerhalb vorgebbarer Zeitspannen Meßinformationen des Sensors auswertet.

Ein großer Freiraum bei der Wahl eines geeigneten Ortes für den Sensor kann dadurch erreicht werden, dass der Sensor im Bereich eines Pflasters angeordnet ist.

Ein schnelles Positionieren des Sensors wird dadurch unterstützt, dass der Sensor im Bereich eines Clips angeordnet ist.

Der Sensor kann an jeder gut durchbluteten Stelle des Körpers angebracht werden. Als sehr gute Stellen sind unter anderem die Ohrläppchen, die Fingerkuppen, die Schläfen und die Stirnfläche sowie der Bereich der Nase zu nennen. Um die Anwendbarkeit und den Tragekomfort deutlich zu erhöhen, kann ein transkutaner Sensor in bevorzugter Form in die Stirnstütze einer Maske für die Beatmungs- und Schlaftherapie eingebettet sein.

Eine ebenfalls bevorzugte Position stellen die Schläfen dar, wobei zur Positionierung entweder die Stirnstütze oder die Bandagen für die Fixierung der gesamten Maske verwendet werden können. Dazu kann der Sensor auf zwei Achsen (x,y) positioniert werden Auch ist eine Integration des Sensors in die Maskenwulst realisierbar.

Das Beatmungsgerät ist geeignet zur Langzeitbeatmung von sehr kleinen Frühgeborenen, von Neugeborenen und von Kindern mit einem Gewicht bis zu 30 Kilogramm und von Erwachsenen. Das Gerät verfügt beispielsweise über Basis-Beatmungsformen CPAP, IPPV/IMV, SIPPV und SIMV und PSV Modi. Weiterhin kann über die Funktion Volumenlimit das abgegebene Tidalvolumen begrenzt werden. In den assistierten Beatmungsformen steht eine volumengesteuerte Tidalvolumengarantie zur Verfügung. Der exakte, patientennah platzierbare (Hitz-draht-)Flowsensor ermöglicht die automatische Nachführung der Triggerempfindlichkeit relativ zum Tidalvolumen des Patienten (VT-Triggeradaption). Das äußerst leistungsfähige, integrierte Hochfrequenz-Modul HFO arbeitet nach dem Membranprinzip, der Frequenzbereich liegt zwischen 5 und 20 Hertz. Die Steuerung der Amplitude erfolgt geregelt und kompensiert im Regelbereich Leckagen und Complianceänderungen. Die möglichst einfache und intuitive Bedienung des Gerätes erfolgt wahlweise über ein UI (3) oder über einen Einstellknopf. Zur optimalen ergonomischen Anpassung an die Platzverhältnisse auf der Station kann das UI abgenommen und unmittelbar an einer Wärmetherapieeinheit oder einem Bett befestigt werden. Alle wesentlichen Einstellungen, Messwerte, Alarmgrenzen und graphischen Informationen wie Kurven und Schleifen stehen auf einen Blick zur Verfügung. Das UI kann vom Anwender nach den eigenen Bedürfnissen konfiguriert werden. So sind die Anzahl von Kurven und Schleifen sowie die angezeigten Messwerte frei wählbar. Die manuelle Regelung des inspiratorischen Sauerstoffs (FiO2) bei Patienten wie beispielsweise Frühgeborenen, die mit Sauer-stoff versorgt werden, ist oft kompliziert und zeitaufwendig.

Der erfindungsgemäße Algorithmus zur automatisierten Sauerstoffsteuerung bei Patienten ist in das Beatmungsgerät integriert. Um die Bedienung des für den Anwender so einfach und intuitiv wie möglich zu machen, wurden der Steueralgorithmus und die Pulsoximetriemessung in das Beatmungsgerät integriert. Die gesamte Bedienung einschließlich Visualisierung der Messdaten und Alarmeinstellungen erfolgt über die Anzeige (Bedienoberfläche UI) des Beatmungsgerätes. Der Anwender kann auf einen Blick den aktuellen graphisch dargestellten Patientenstatus ermitteln. Der erfindungsgemäße Algorithmus nimmt dem Kliniker die Routineanpassung des inspiratorischen Sauerstoffs im Atemgas ab (FiO2), indem es kontinuierlich den Bedarf und Zustand des Patienten überwacht und die Geräteeinstellungen entsprechend anpasst. Somit wird der Anwender von Routineaufgaben entlastet. Es besteht jederzeit die Möglichkeit, die automatische Steuerung abzuschalten, um den Sauerstoffgehalt manuell zu regeln. Der erfindungsgemäße Algorithmus kann die Sauerstoffversorgung bei Patienten, die mechanisch oder über eine nasale CPAP-Atemmaske beatmet werden, verbessern und gleichzeitig die mit manueller Einstellung verbundene Arbeitsbelastung reduzieren.

Das Pulsoximeter mit Sensor wird beispielsweise an die Serielle-Schnittstelle des Beatmungsgerätes angeschlossen. Ist die "Closed Loop" Funktion aktiviert, so wird der FiO2-Wert während einer Beatmung automatisch gesetzt, gemessen und angezeigt.

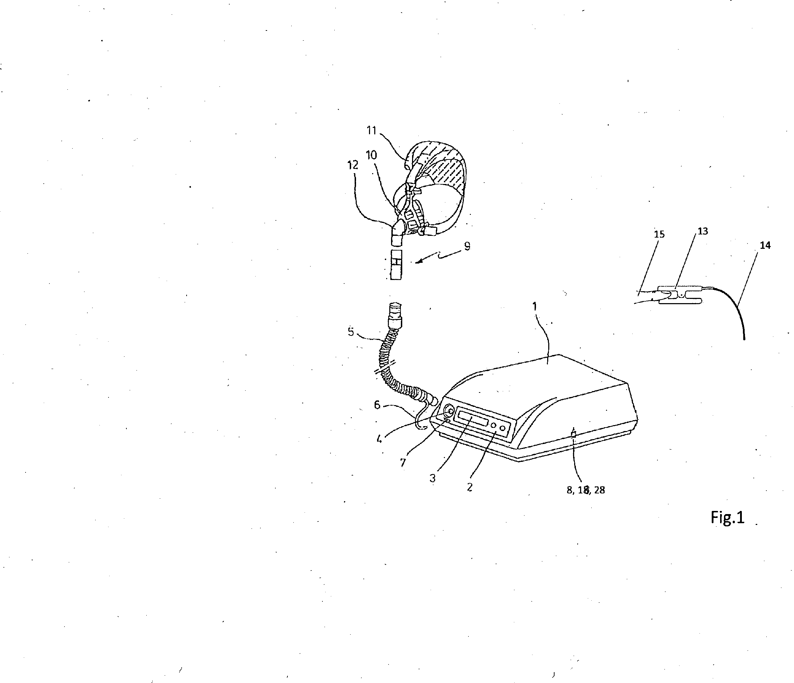

Fig. 1 zeigt den grundsätzlichen Aufbau eines Beatmungsgerätes (1). Im Bereich eines Gerätegehäuses mit Bedienfeld (2) sowie Anzeige (3) ist in einem Geräteinnenraum eine Atemgasquelle angeordnet. Die Atemgasquelle kann als Lüfter und/oder Atemgas- oder Sauerstoffleitung oder anderweitig ausgebildet sein. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Das Gerät weist zumindest eine Schnittstelle (8,18,28) auf. Ein Anfeuchter kann adaptiert werden. Die Atemgasquelle kann als Lüfter und/oder Ventil oder auch als integrierte Hochfrequenz-Modul (HFO), welches nach dem Membranprinzip arbeitet, ausgebildet sein (der typische Frequenzbereich liegt dann zwischen 3 und 30 Hertz).

Im Bereich einer dem Gerätegehäuse (1) abgewandten Ausdehnung des Verbindungsschlauches (5) ist beispielsweise ein Ausatmungselement (9) angeordnet. Ebenfalls kann ein Ausatemventil verwendet werden. Auch ist eine Zweischlauchbeatmung möglich.

Beatmungsgerät zur Beatmung eines Patienten mit FiO2 Vorgabewerten, einer Steuereinrichtung, einer Atemgasquelle, einer Sauerstoffquelle, einer Anzeige (3) und Bedienelementen (2) sowie zumindest einer Schnittstelle (8,18,28) über die ein SPO2-Sensor an das Beatmungsgerät angeschlossen ist zur Bestimmung einer Sauerstoffsättigung des Patienten, wobei die Steuereinrichtung, die Atemgasquelle und die Sauerstoffquelle zur Vorgabe einer definierten FiO2 ansteuert, wobei über die Bedienelemente (2) oder eine Schnittstelle Zielwerte für die SpO2 und/oder FiO2 vorgebbar sind dadurch gekennzeichnet, dass die Steuereinheit aktuelle Meßwerte und/oder Zielwerte für SpO2 auf der Anzeige darstellt und aktuelle Meßwerte und/oder Vorgabewerte für FiO2 auf der Anzeige darstellt.

Die Steuereinrichtung steuert die Atemgasquelle und die Sauerstoffquelle zur Vorgabe einer definierten FiO2 an, die Steuereinheit berücksichtigt aktuelle Meßwerte und/oder Zielwerte für SpO2 und aktuelle Meßwerte und/oder Vorgabewerte für FiO2 für die Regelung der FiO2 zumindest zeitweise.

Die Steuereinheit stellt aktuelle Meßwerte für SpO2 auf der Anzeige darstellt und aktuelle Meßwerte und/oder Vorgabewerte für FiO2 auf der Anzeige in Kurvenform als Verlaufskurven dar.

Das Beatmungsgerät weist auch einen Speicher für zumindest die Messwerte, Zielwerte und die Vorgabewerte auf.

Die Verlaufskurven für SpO2 und FiO2 werden untereinander darstellt.

Am Ende der Verlaufskurve für SpO2 wird der aktuelle Meßwert (31) für SpO2 dargestellt. Am Ende der Verlaufskurve für FiO2 wird der aktuelle Meßwert für FiO2 dargestellt.

Am Ende der Verlaufskurve ist der aktuelle Meßwert für FiO2 und/oder der aktuelle Meßwert (31) für SpO2 mit einem Pfeilsymbol (52) hinterlegt, wobei die Ausrichtung des Pfeils (52) dabei dynamisch anhand der vergangenen Meßwerte angepasst wird.

Für SpO2 wird ein Zielwert visualisiert und Grenzwerte visualisiert werden, wobei die Grenzwerte farblich oder anders visualisiert werden als die Zielwerte.

Fig. 1 zeigt darüber hinaus ein als Beatmungsmaske (10) ausgebildetes Patienten-Interface (10), das als Nasalmaske realisiert ist. Eine Fixierung im Bereich eines Kopfes eines Patienten kann über eine Kopfhaube (11) erfolgen. Im Bereich ihrer dem Verbindungsschlauch (5) zugewandten Ausdehnung weist das Patienten-Interface (10) ein Kupplungselement (12) auf. Das Patienten-Interface (10) kann auch als Nasenstopfen, Nasenbrille oder Tubus ausgebildet sein.

Über die Schnittstelle (8, 18,28) kann der SpO2-Sensor an das Beatmungsgerät angeschlossen werden. Die Schnittstellen können kabelgebunden, als Infrarot-Schnittstelle, als Bluetooth-Schnittstelle oder USB realisiert sein.

Die FiO2 Vorgabewerte werden derart umgesetzt, dass die Steuereinheit Gas der Atemgasquelle (beispielsweise Umgebungsluft), mit dem Sauerstoff der Sauerstoffquelle, in der Gasmischeinheit derart bereitstellt, dass Atemgas entsprechend dem FiO2 Vorgabewert für die Beatmung bereitgestellt wird.

Im Bereich eines Gerätegehäuses kann ein Sauerstoffzuschaltventil adaptiert werden. Das Sauerstoffzuschaltventil kann auch im Beatmungsgerät angeordnet sein und insofern Teil der Atemgasquelle sein. Es ist denkbar das Atemgas zusätzlich mit Sauerstoff anzureichern, um die Patientenversorgung zu verbessern.

Die Schnittstelle (8,18,28) ist vorgesehen für eine Verbindung mit dem Sensor (13), welcher zur Messung von zumindest SpO2 und/oder der Pulsrate oder anderen Blutgaswerten vorgesehen ist. Die Verbindung erfolgt über ein Kabel (14) oder kabellos. Der Sensor wird am Patienten (15) beispielsweise am Finger platziert.

Eine der Schnittstellen (8,18,28) kann eine Schnittstelle zu Drittgeräten und Informations-Management-Systemen beispielsweise zur Aufnahme von Speichermedien, zur Verbindung mit einem EKG, EEG, Drucker, Defibrillator etc. vorgesehen werden.

Über ein Modem oder eine andere Schnittstelle (8,18,28) können ebenfalls aufgezeichnete Daten, wie Trends der SpO2, außerordentliche Ereignisse, Warnmeldungen oder ähnliches wie Auffälligkeiten, Betriebsstunden bei Bedarf oder automatisch übermittelt werden. Die Daten können von der Gegenstelle gespeichert, analysiert oder visualisiert werden. Beispielsweise ist die Gegenstelle in einem Überwachungsraum vorgesehen, wo Anwender eine Vielzahl von erfindungsgemäßen Beatmungsgeräten überwachen und nötigenfalls auch aus der Ferne über die Schnittstelle steuern können.

Um den Spareffekt zu erzielen, könnte man je nach Sauerstoffsättigung die Sauerstoffzufuhr generell oder in der anfänglichen Einatmungsphase steuern und applizieren. Damit und mit der Überwachung des FiO2 (FiO₂ ist die Abkürzung für die inspiratorische Sauerstofffraktion) und der daraus resultierenden Berechnung der Differenz von Ist- und Ziel-Wert ist eine abhängige O2-Gabe möglich.

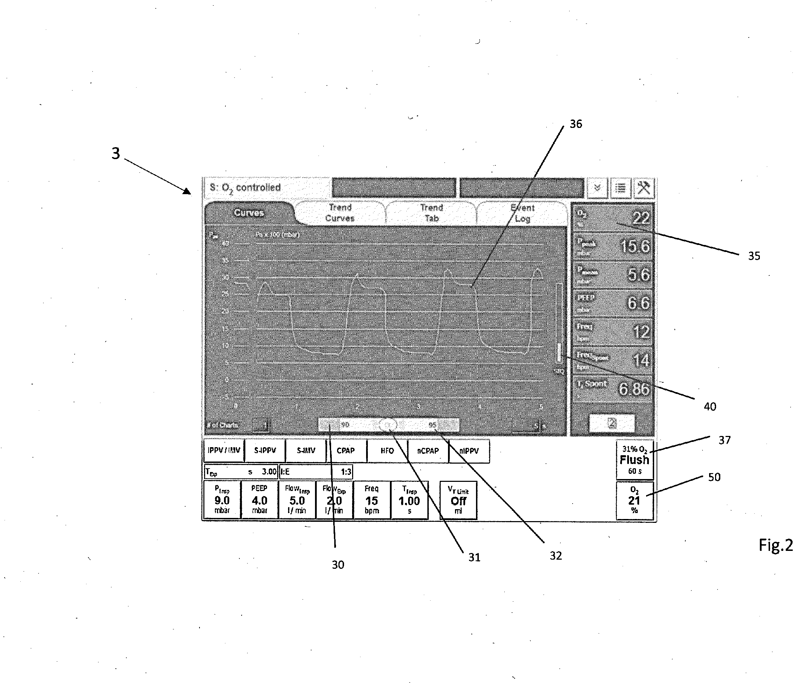

Fig. 2 zeigt die Anzeige (3) oder das UI mit Feldern für die Darstellung von Kurven (36) der Meßwerte, mit den Einstellwerten (35) und mit einem Index für die Signalqualität (40) des SpO2-Signales.

Die gesamte Bedienung einschließlich Visualisierung der Messdaten und Alarmeinstellungen erfolgt über die (Bedienoberfläche der) Anzeige des Beatmungsgerätes.

Der gemessene SpO2-Wert (31) wird auf der Anzeige (3) angezeigt, beispielsweise ganzzahlig ohne Nachkommastelle. Die Signalqualität (40) der SpO2-Messung wird als Grafik angezeigt. Eingestellte Werte (35) werden in einem Randbereich der Anzeige visualisiert.

Der aktuelle Meßwert für SpO2 (31) wird in einer SpO2-Grafik (30) dargestellt. Hier werden auch vorgegebene Grenzwerte (32) visualisiert. Meßwerte für SpO2, die in dem voreingestellten Bereich liegen, werden farblich, z.b. grün oder gelb, hinterlegt. Werte die außerhalb von dem voreingestellten Bereich liegen, werden bevorzugt rot hinterlegt.

Ein Feld (50) visualisiert die Anwendung einer kurzzeitigen (einstellbar) erhöhten SauerstoffKonzentration im Atemgas FiO2 (einstellbar, von beispielsweise 21 - 100%). Im Falle einer Touchanzeige (3) wird durch Anwenderauswahl von Feld (37) kurzzeitig die erhöhte Sauer-stoff-Konzentration appliziert.

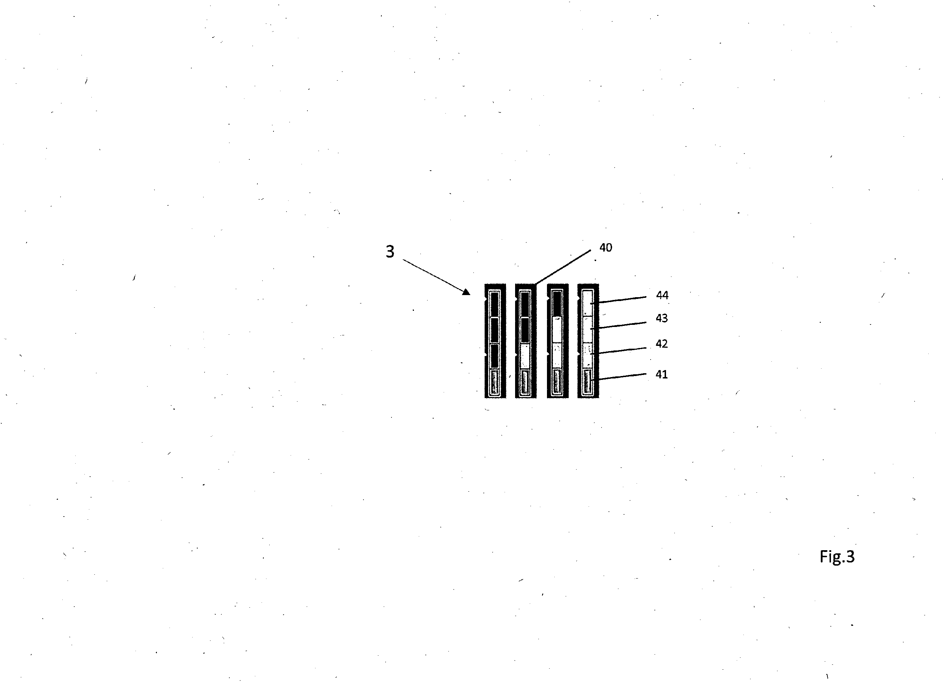

Fig. 3 zeigt die Darstellung der Signalqualität (40) des SpO2-Signals als Grafik. Der Prozentwert wird als Balkengrafik angezeigt, mit einer Unterteilung in 4 Teilbereiche: 41(0-33%), 42 (33% - 75%), 43 (75%-90%), 44 (90%-100%). Die Teilbereiche weisen zudem unterschiedliche Farben auf z.b. Farben: 41= rot, 42 = gelb, 43 = grün, 44 = grün.

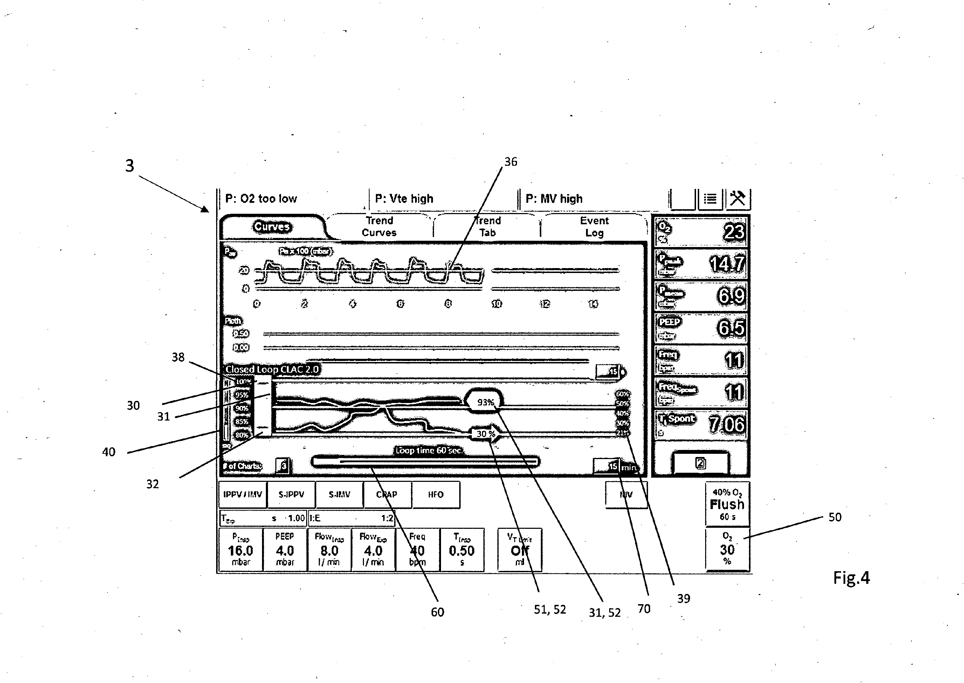

Fig. 4 zeigt die Anzeige (3) oder das UI mit abgewandelten Feldern. Der aktuelle Meßwert für SpO2 (31) wird in einer SpO2-Grafik (30) dargestellt. Hier werden auch vorgegebene Grenzwerte (32) visualisiert. Der aktuelle Meßwert für SpO2 (31) wird jedoch als Verlaufswert beispielsweise in Kurvenform dargestellt, wobei der aktuelle Wert am Ende der Kurve als %-Wert dargestellt ist.

Der Wert der aktuellen FiO2 (51) wird ebenfalls als Verlaufswert beispielsweise in Kurvenform dargestellt, wobei der aktuelle Wert am Ende der Kurve als %-Wert dargestellt ist. Bevorzugt verläuft die Kurve für FiO2 unterhalb der Kurve für SpO2. In einer beispielhaften Darstellung beginnen die Messwert links und verlaufen nach rechts. Bevorzugt skaliert sich die Kurvendarstellung für SpO2 und/oder FiO2 selbst anhand der Messwerte. Bevorzugt wird die Kurvendarstellung für SpO2 und/oder FiO2 für die gesamte Meßzeit (70) durchgeführt.

Der Wert der aktuellen FiO2 (51) und/oder der aktuelle Meßwert für SpO2 (31) können hinterlegt sein mit einem (Pfeil)symbol (52). Der Pfeil oder das Symbol gibt dem Arzt oder Anwender Information über zu erwartende Änderung der FiO2 in der nachfolgenden Regelperiode.

Der Anwender weiss somit frühzeitig, ob eine Intervention notwendig ist oder ob der automatische Ablauf den FiO2 ausreichend genau regelt.

Der Pfeil (52) wird dabei dynamisch anhand der vergangenen Werte für SpO2 bzw. FiO2 in seiner Ausrichtung angepasst. Bevorzugt ist der Pfeil (52) waagerecht, was bedeutet, dass die Messwerte für SpO2 sich in dem vorgesehenen therapeutischen Bereich befinden und eine Änderung der FiO2 nicht erfolgen muss. Weist der jeweilige Pfeil (52) nach unten, dann ist der Trend der vergangenen Werte für SpO2 aufsteigend, hin zu höheren Meßwerten für SpO2. Entsprechend würde die Vorgabe für FiO2 für die nachfolgende Regelperiode (60) erniedrigt werden. Weist der jeweilige Pfeil (52) nach oben, dann ist der Trend der vergangenen Werte für SpO2 absteigend, hin zu geringeren Meßwerten für SpO2. Entsprechend würde die Vorgabe für FiO2 für die nachfolgende Regelperiode (60) erhöht werden.

Die automatische Regelung umfasst beispielsweise auch bei einem Abfall der SpO2 eine Verkürzung der Regelperiode (60) und eine Erhöhung der FiO2 für die nachfolgende Regelperiode (60). Gleichwohl ist auch vorgesehen, dass der Anwender diese Automatismen deaktiviert oder individuell konfiguriert.

Dabei ist eine Skala (39) für die FiO2 am rechten Rand der Kurvendarstellung angeordnet und eine Skala (38) für die SpO2 am linken Rand der Kurvendarstellung.

Die Anzeigezeit der Kurve bzw. die Meßzeit (70) ist konfigurierbar, wobei beispielsweise Zeiträume von 5 min bis 12 h definierbar sind. Vorliegend sind 15 min eingestellt.

Ein Zeitgraph (60) für die Regelperiode (60) verläuft dynamisch, beispielsweise in Abhängigkeit von der eingestellten Therapiedauer. Dabei wird die bereits vergangene Therapiedauer farblich anders dargestellt, als die verbleibende Therapiedauer,

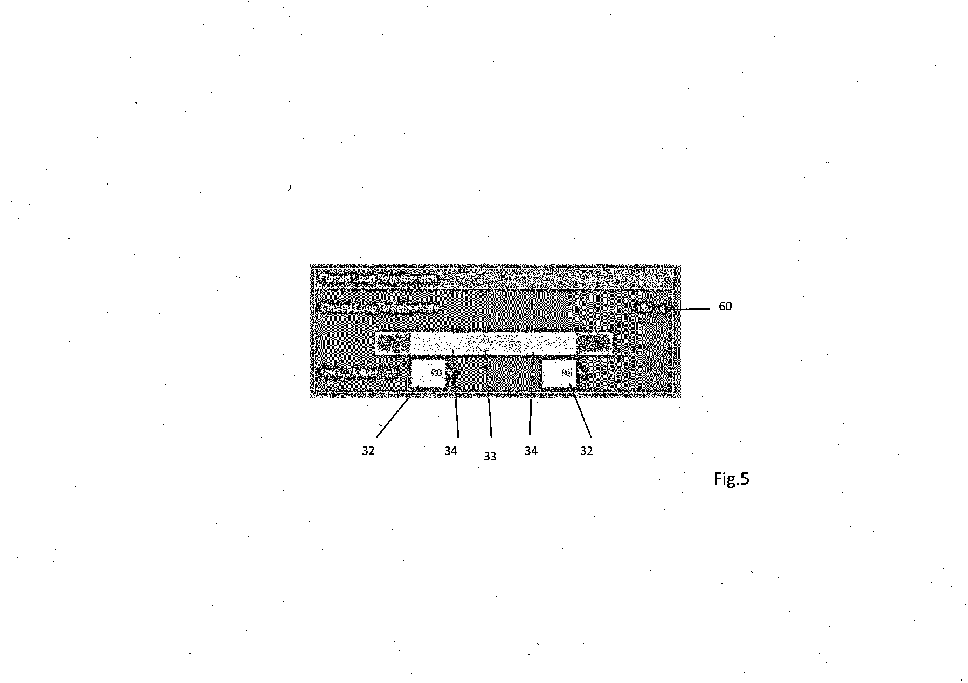

Fig.5 zeigt das Einstellen des SpO2 Zielbereichs durch einen Anwender. Der Zielbereich weist einen minimalen und maximalen Grenzwert (32) auf. Dieser Wertebereich wird unterteilt in beispielsweise drei Bereiche. Diese drei Bereiche beinhalten den eigentlichen Zielbereich (grüner) Bereich (33) mit den Bereichen der oberen (34) und unteren (34) gelber Bereich Normoxämie.

Das Einstellen der Regelperiode ist ebenfalls möglich. Eingestellt werden können 10 - 600 s (mit einer Schrittweite von 5 - 30 s). Bevorzugt ist es im Bereich 30 - 180 Sekunden. In Abhängigkeit der Krankheitsbilder gibt es Patienten, die auf Entsättigung unterschiedlich schnell reagieren. Deshalb ist der mögliche Bereich weit gewählt. Im Betrieb ist die Regelperiode auch jederzeit anzupassen, ohne die Beatmung unterbrechen zu müssen.

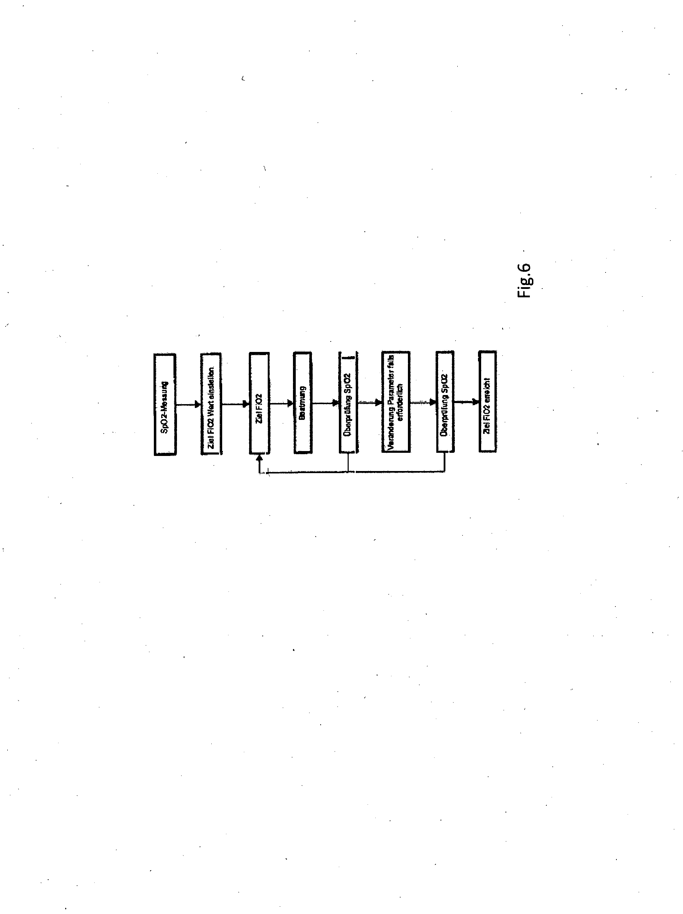

Die Durchführung des Verfahrens wird in Fig.6 beispielhaft erläutert.

Dargestellt ist ein Beatmungsgerät zur Beatmung eines Patienten mit Vorgabewerten einer Sauerstoffkonzentration (FiO2) im Atemgas, mit einer programmierbaren Steuereinrichtung, einer Atemgasquelle, einer Sauerstoffquelle, einer Gasmischeinheit, einer Anzeige (3) und Bedienelementen (2) sowie zumindest einer Schnittstelle (8,18,28) und einem Sauerstoff-Sensor zur Bestimmung einer Sauerstoffsättigung (SpO2) des Patienten, wobei die programmierbare Steuereinrichtung zum Durchführen des folgenden Verfahrens eingerichtet ist:
Vorgabe von einem Zielwert oder Grenzwerten für die SpO2 und
Vorgabe einer FiO2 und
Vorgabe einer Regelperiode (60) für die Beatmung mit der FiO2 und
Beatmung mit einer Sauerstoffkonzentration (FiO2) im Atemgas entsprechend der Vorgabe für die Zeitdauer der Regelperiode (60)
Ermittlung von aktuellen Meßwerten für SpO2 während der Zeitdauer der Regelperiode (60) Darstellung der Meßwerte für SpO2 auf der Anzeige und
Darstellung der Vorgabe für FiO2 auf der Anzeige.

Das Verfahren beinhaltet auch, dass die Beatmung mit einer Sauerstoffkonzentration (FiO2) im Atemgas entsprechend der Vorgabe für die Dauer der Regelperiode (60) erfolgt und dabei Meßwerte der SpO2 während der Beatmung aufgezeichnet werden und analysiert wird, ob die Meßwerte der SpO2 dem Zielwert entsprechen und bei einer Abweichung der Meßwerte der SpO2 vom dem Zielwert für die nachfolgende Regelperiode (60) ein neuer Vorgabewert für FiO2 angewendet wird.

Bei einer Standardbeatmung mit Vorgabe von beispielsweise FiO₂ = 30%, beträgt die Regelperiode (60) 60 sec. und der Grenzbereich für SpO2 ist 88% - 94%, die Alarmgrenzen sind weit gestellt.

Start mit SpO₂ 91% die nach einer Anwendervorgabe in Richtung von 100% erhöht werden soll. Eine manuelle Änderung der FiO₂ 30% -> auf 60% erfolgt durch den Anwender (jederzeit manueller Eingriff durch den Anwender möglich).
- eine Reduktion der FiO2 um 15% erfolgt nach der Regelperiode, bei weiterhin 100% SpO2
- Reduktion 8% nach der Regelperiode, bei weiterhin 100%
- Reduktion 4% nach der Regelperiode, bei weiterhin 100%
- Die Regelung hat den FiO₂ Wert zu diesem Zeitpunkt bereits auf 33% zurückgeführt.

Eine Reduktion der SpO₂ auf 91% ist erfolgt.

Bei einer Entsättigung im normalen Regelbereich und bis zu beispielsweise minimal 70% SpO2:
Bei einem Abfall von 5% SpO₂ wird die FiO₂ für die nächste Regelperiode um 5% erhöht.
Bei einem Abfall von >5-10% SpO₂ soll sie die FiO₂ um 8% erhöhen
Bei einem Abfall von >10% SpO₂ soll sie die FiO₂ um 10% erhöhen
Wenn der Abfall des SpO₂ Wertes aufgehalten wird, der normale Regelbereich gelb / grün gelb erreicht wird, tritt die normale Regelung wieder in Kraft.

Bis zu diesem Zeitpunkt wird immer wiederkehrend eine Erhöhung der FiO₂ vorgenommen, in der Höhe des initial ermittelten Verstell-Wertes ist beispielsweise 5%, 8% oder 10%. Mit dem vorgewählten Zeitfenster für den Regelbereich von 30 - 180 sec.

Bei einer Standardbeatmung: beispielsweise FiO₂ set = 30%, Regelbereich auf 60 sec. 88% - 94% SpO2, Alarmgrenzen weit gestellt
SpO₂ Abfall von 91% -> 84%, 7% Abfall der Sättigung
Therapie Regelzeit bleibt bei 60 sec., FiO2 Erhöhung erfolgt nach Ablauf dieser Zeit immer mit 8 %, bis die SpO2 wieder im "normalen" Bereich ist
SpO2 erhöht auf 89%, erlöschen der Alarme, Aussetzen der "Dynamische O2 Regelung"

Schnelle Entsättigung von beispielsweise unter 70%:
Es entsteht ein plötzlicher SpO₂ Abfall unter die Grenze von 70% SpO₂ (unabhängig von Alarm Einstellung und auch den vorgewählten Regelbereich- Definitionen). Die Reaktion erfolgt in-nerhalb von 10 sec.
Roter Alarm wird ausgelöst - normale Regelung ausgesetzt
Zusätzlich zum roten Alarm wird der FiO₂ Button rot Blinken (Alarm Management Eskalation) Alle Erhöhungen der FiO2 erfolgen spätestens nach 30 sec. Die normale Regelzeit Einstellung wird ignoriert.
Unterschreiten von 70% führt automatisch zum höchst möglichen Erhöhungsdelta -> aktuelles FiO₂ + beispielsweise 10%
Diese Erhöhung wird nur dann beendet, wenn die SpO₂ Messwerte wieder im gelb / grün / gelben Bereich liegen, also alle 10 sec. FiO₂ + 10%, bis FiO₂ 100% erreicht ist.

Standardbeatmung, beispielsweise FiO₂ set = 30%, CLAC auf 60 sec. 88% - 94%, Alarmgrenzen weit gestellt.
SpO₂ von 91% -> 68%, 23% Abfall der Sättigung
Auslösen Hoch Prioritäts "Alarm SpO₂ zu niedrig - Notfallregelung aktiv", sowie rotes Blinken des FiO₂ Buttons, da schnell hochgeregelt wird.

Therapie Regelzeit ändert sich auf 30 sec., die FiO₂ Erhöhung erfolgt nach Ablauf dieser Zeit immer mit 10%, bis die SpO₂ wieder im "normalen" Bereich landet.

SpO2 erhöht auf 91%, erlöschen der Alarme, Aussetzen der "Alarm SpO₂ zu niedrig - Notfallregelung aktiv", Blinken stoppt.

Wird jetzt SpO₂ von 91% -> 100% simuliert, führt das zur Notfall Regelung Reduktion.
1. Zeitraum (10 Sek), akzeptierte SpO₂ Messergebnisse zwischen 95% und 100%
2. Zeitraum (10 Sek), akzeptierte SpO₂ Messergebnisse zwischen 98% und 100%
3. Zeitraum (10 Sek - 160 Sek) abhängig vom aktuellen Therapiezeitraum, akzeptierte SpO₂ Messergebnisse 99% - 100%, die 10 Sekunden dauern jetzt bis die eingestellte Therapiezeit durchlaufen wurde, also bis zu 180 Sekunden insgesamt.

Ist der letzte Zeitraum positiv durchlaufen worden, so soll die Regelung die Hälfte der vorher manuell erhöhten Differenz zurück regeln (Notfallregelung).

Oder die vorherige Erhöhung basiert auf der Notfallerhöhung (+ 10%), dann gilt auch der Notfall Reduktions-Algorithmus.

Liegt ein Wert nicht für die entsprechende Zeit in diesen Bereichen oder werden die drei Stufen nicht durchlaufen, die Regelung erfolgt so wie immer.

Von außen ist beispielsweise auch vorgebbar, direkt ein Krankheitsmuster oder ein Zielwert einzugeben, wobei dem Gerät je nach Autonomiegrad eine fast vollständige Autonomie über die folgenden Entscheidungsmöglichkeiten gewährt wird. Die Daten welche für die Geräteautonomie nötig sind und als bspw. Einstellungen, Bandbreiten, Minima und Maxima verwendet werden, sind im Gerät abgespeichert und werden bei Bedarf ausgelesen. Daraufhin wird vom Gerät eine Entscheidung bezüglich des Beatmungsverfahrens (druck-/volumenkontrollierte Beatmung) verlangt.

In einem nächsten Schritt wird beispielsweise abgefragt, ob der Patient assistiert, kontrolliert oder assistiert/kontrolliert beatmet werden soll. Nun kann je nach Autonomiegrad von außen oder durch das Gerät entschieden werden, welche Parameter und in welchen Bandbreiten, Maxima und Minima der Beatmungsparameter, eingestellt werden oder ob man sich einer Liste von Strategien bedient. Jede dieser Strategien beinhaltet eine Prioritätenliste von 1 bis N verschiedenen Einstellungen und wird im Rahmen ihrer Bandbreiten abgearbeitet.

Es kann beispielsweise eine Abfrage des momentanen PaCO2-Wertes und Vergleich dieses mit dem Ziel-CO2-Wert erfolgen, so dass eine Entscheidung bezüglich des weiteren Abarbeitens der priorisierten Bandbreite erfolgen kann. Ist die Änderung einer Bandbreite erschöpft, wird der evtl. in der Strategie nachfolgende Parameter so lange geändert bis der Ziel-CO2-Wert für den Patienten optimal eingestellt ist. Bei kompletter Abarbeitung einer Strategie und gleichzeitigem Nicht-Erreichen des Ziel-CO2-Wertes könnte entweder eine neue Strategie, die Einstellung eines neuen Ziel-CO2-Wertes, ein neues Beatmungsverfahren/Kontrollgrößen (druck-/volumenkontrolliert) oder ein neuer Modus (assistierte und/oder kontrollierte Beatmung) eingestellt werden, um den Patientenzustand weiter zu verbessern. Diese Entscheidung kann entweder durch einen Alarm dem Anwender mitgeteilt und/oder gefordert oder durch das Gerät selbstständig ausgeführt werden.

Alternativ kann beispielsweise auch der Zielwert, auf den hingesteuert werden soll, in eine Bandbreite gefasst werden, so dass das Gerät zwar ein Ziel hat, den es gilt zu erreichen. Falls jedoch Strategien ausgeschöpft sein sollten, so können die aktuell erreichten Einstellungen auch vom Gerät als akzeptabel angenommen werden. Der Zielwert sowie die Zielwertbandbreite kann in den Geräteeinstellungen vorgenommen werden.

Ferner ist es beispielsweise möglich, die Änderungsintensität abzustufen und einer Schwellenbildung zu unterziehen. Je nachdem welche Schwelle(n) überschritten wird, wird die Änderungsintensität angepasst.

## Patentansprüche

1. Beatmungsgerät zur Beatmung eines Patienten mit Vorgabewerten einer Sauerstoffkonzentration (FiO2) im Atemgas, mit einer programmierbaren Steuereinrichtung, einer Atemgasquelle, einer Sauerstoffquelle, einer Gasmischeinheit, einer Anzeige (3) und Bedienelementen (2) sowie zumindest einer Schnittstelle (8,18,28) und einem Sauerstoff-Sensor zur Bestimmung einer Sauerstoffsättigung (SpO2) des Patienten, wobei die programmierbare Steuereinrichtung zum Durchführen des folgenden Verfahrens eingerichtet ist:
• Vorgabe von einem Zielwert oder von Grenzwerten für die SpO2 und
• Vorgabe einer FiO2 im Atemgas und
• Vorgabe einer Regelperiode (60) für die Beatmung mit der FiO2 und
• Beatmung mit einer Sauerstoffkonzentration (FiO2) im Atemgas entsprechend der Vorgabe für die Zeitdauer der Regelperiode (60)
• Ermittlung von aktuellen Meßwerten für SpO2 während der Zeitdauer der Regelperiode (60)
• Darstellung der Meßwerte für SpO2 auf der Anzeige und
• Darstellung der Vorgabe für FiO2 auf der Anzeige,
wobei die Signalqualität (40) des SpO2-Signals, beispielsweise anhand der Lichtintensität, ermittelt und auf der Anzeige (3) derart visualisiert wird, dass eine gute Signalqualität (40) visuell anders dargestellt wird als eine schlechte Signalqualität,
**dadurch gekennzeichnet, dass** das Verfahren beinhaltet, dass die Beatmung mit einer Sauerstoffkonzentration (FiO2) im Atemgas entsprechend der Vorgabe für die Dauer der Regelperiode (60) erfolgt und dabei Meßwerte der SpO2 während der Beatmung aufgezeichnet werden und analysiert wird, ob die Meßwerte der SpO2 dem Zielwert entsprechen und bei einer definierten Abweichung der Meßwerte der SpO2 vom Zielwert für die nachfolgende Regelperiode (60) ein neuer Vorgabewert für FiO2 berechnet und/oder angewendet wird und wobei basierend auf der Abweichung der Messwerte für SpO2 von dem Zielwert eine Veränderung für den FiO2 berechnet wird und auf der Anzeige ein Symbol (52) dargestellt wird, wobei das Symbol (52) dem Betrachter eine Information darüber gibt, ob der nachfolgende Vorgabewert für FiO2 - im Vergleich zu dem aktuellen FiO2-Wert - gleichbleibt oder verändert wird.

2. Beatmungsgerät nach zumindest einem der vorhergehenden Ansprüche, **dadurch gekennzeichnet, dass** das Verfahren beinhaltet, dass die Dauer der Regelperiode (60) entsprechend der Abweichung der Meßwerte der SpO2 vom Zielwert für die nachfolgende Regelperiode (60) angepasst wird.

3. Beatmungsgerät nach zumindest einem der vorhergehenden Ansprüche, **dadurch gekennzeichnet, dass** das Verfahren beinhaltet, dass die Regelperiode im Bereich 20 - 240 Sekunden vorgebbar ist oder angepasst wird.

4. Beatmungsgerät nach zumindest einem der vorhergehenden Ansprüche, **dadurch gekennzeichnet, dass** das Verfahren beinhaltet, dass auf der Anzeige ein Zeitgraph für die Regelperiode (60) dynamisch dargestellt wird.

5. Beatmungsgerät nach Anspruch 1 oder 2, **dadurch gekennzeichnet, dass** das Verfahren beinhaltet, dass Meßwerte für SpO2 und/oder Vorgabewerte für FiO2 in einer Verlaufsform chronologisch auf der Anzeige dargestellt werden.

6. Beatmungsgerät nach zumindest einem der vorhergehenden Ansprüche, **dadurch gekennzeichnet, dass** das Verfahren beinhaltet, dass Vorgabewerte für FiO2 und/oder Meßwerte für SpO2 in Form von Verlaufskurven dargestellt werden.

7. Beatmungsgerät nach zumindest einem der vorhergehenden Ansprüche, **dadurch gekennzeichnet, dass** das Verfahren beinhaltet, dass die Verlaufskurven für SpO2 und FiO2 untereinander darstellt werden.

8. Beatmungsgerät nach zumindest einem der vorhergehenden Ansprüche, **dadurch gekennzeichnet, dass** das Verfahren beinhaltet, dass am Ende der Verlaufskurve für SpO2 der aktuelle Meßwert (31) für SpO2 dargestellt wird und/oder dass am Ende der Verlaufskurve für FiO2 der aktuelle Vorgabewert für FiO2 dargestellt wird.

9. Beatmungsgerät nach zumindest einem der vorhergehenden Ansprüche, **dadurch gekennzeichnet, dass** das Verfahren beinhaltet, dass am Ende der Verlaufskurve der aktuelle Vorgabewert für FiO2 und/oder der aktuelle Meßwert (31) für SpO2 mit einem Symbol (52) hinterlegt ist, wobei die Ausrichtung des Symbols (52) dabei dynamisch anhand der vergangenen Meßwerte (31) angepasst wird.

10. Beatmungsgerät nach zumindest einem der vorhergehenden Ansprüche, **dadurch gekennzeichnet, dass** das Verfahren beinhaltet, dass der aktuelle Messwert für SpO2 auf der Anzeige gemeinsam mit einem Symbol (52) dargestellt wird, wobei die Ausrichtung des Symbols (52) dabei dynamisch derart angepasst wird, dass die Ausrichtung des Symbols dem Betrachter eine Information darüber gibt, ob der nachfolgende Vorgabewert für FiO2 gleichbleibt oder verändert wird.

11. Beatmungsgerät nach zumindest einem der vorhergehenden Ansprüche, **dadurch gekennzeichnet, dass** das Verfahren beinhaltet, dass bei einem definierten Abfall der SpO2 eine Verkürzung der aktuellen Regelperiode (60) und/oder eine Erhöhung der FiO2 für die nachfolgende Regelperiode (60) erfolgt.

12. Beatmungsgerät nach zumindest einem der vorhergehenden Ansprüche, **dadurch gekennzeichnet, dass** das Verfahren beinhaltet, dass für SpO2 ein Zielwert visualisiert wird und Grenzwerte visualisiert werden, wobei die Grenzwerte farblich hervorgehoben oder anders visualisiert werden als die Zielwerte.

## Claims

1. A ventilator for ventilating a patient with setpoint values of an oxygen concentration (FiO2) in the respiratory gas, having a programmable control apparatus, a respiratory gas source, an oxygen source, a gas mixing unit, a display (3) and operating elements (2), as well as at least one interface (8, 18, 28) and an oxygen sensor for determining an oxygen saturation (SpO2) of the patient, wherein the programmable control apparatus is configured to perform the following method:
• Specifying a target value or limit values for the SpO2, and
• Specifying an FiO2 **in** the respiratory gas, and
• Specifying a closed-loop control period (60) for the ventilation with the FiO2, and
• Ventilating with an oxygen concentration (FiO2) **in** the respiratory gas corresponding to the specification for the time duration of the closed-loop control period (60)
• Ascertaining current measured values for SpO2 during the time duration of the closed-loop control period (60)
• Showing the measured values for SpO2 on the display, and
• Showing the specification for FiO2 on the display,
wherein the signal quality (40) of the SpO2 signal is ascertained, for example based on the light intensity, and visualized on the display (3) such that a good signal quality (40) is shown visually differently from a poor signal quality,
**characterized in that** the method includes the ventilation taking place with an oxygen concentration (FiO2) in the respiratory gas corresponding to the specification for the duration of the closed-loop control period (60) and, in the process, measured values of the SpO2 being recorded during the ventilation and it being analyzed whether the measured values of the SpO2 correspond to the target value and, in the case of a defined deviation of the measured values of the SpO2 from the target value, a new setpoint value for FiO2 is calculated and/or applied for the following closed-loop control period (60) and wherein, based on the deviation of the measured values for SpO2 from the target value, a change is calculated for the FiO2 and a symbol (52) is shown on the display, wherein the symbol (52) gives the viewer information about whether the following setpoint value for FiO2 - compared to the current FiO2 value - stays the same or is changed.

2. The ventilator according to at least one of the preceding claims, **characterized in that** the method includes the duration of the closed-loop control period (60) being adapted for the following closed-loop control period (60) corresponding to the deviation of the measured values of SpO2 from the target value.

3. The ventilator according to at least one of the preceding claims, **characterized in that** the method includes the closed-loop control period being specifiable or adapted in the range of 20 - 240 seconds.

4. The ventilator according to at least one of the preceding claims, **characterized in that** the method includes a time graph for the closed-loop control period (60) being dynamically shown on the display.

5. The ventilator according to claim 1 or 2, **characterized in that** the method includes measured values for SpO2 and/or setpoint values for FiO2 being shown chronologically on the display in the form of a curve.

6. The ventilator according to at least one of the preceding claims, **characterized in that** the method includes setpoint values for FiO2 and/or measured values for SpO2 being shown in the form of trend curves.

7. The ventilator according to at least one of the preceding claims, **characterized in that** the method includes the trend curves for SpO2 and FiO2 being shown one below the other.

8. The ventilator according to at least one of the preceding claims, **characterized in that** the method includes the current measured value (31) for SpO2 being shown at the end of the trend curve for SpO2 and/or the current setpoint value for FiO2 being shown at the end of the trend curve for FiO2.

9. The ventilator according to at least one of the preceding claims, **characterized in that** the method includes a symbol (52) being placed behind the current setpoint value for FiO2 and/or the current measured value (31) for SpO2 at the end of the trend curve, wherein the orientation of the symbol (52) is adapted dynamically based on the past measured values (31).

10. The ventilator according to at least one of the preceding claims, **characterized in that** the method includes the current measured value for SpO2 being shown on the display together with a symbol (52), wherein the orientation of the symbol (52) is dynamically adapted such that the orientation of the symbol gives the viewer information about whether the following setpoint value for FiO2 stays the same or is changed.

11. The ventilator according to at least one of the preceding claims, **characterized in that** the method includes, in the case of a defined decrease in the SpO2, a shortening of the current closed-loop control period (60) and/or an increase in the FiO2 for the following closed-loop control period (60) taking place.

12. The ventilator according to at least one of the preceding claims, **characterized in that** the method includes a target value being visualized and limit values being visualized for SpO2, wherein limit values are emphasized in color or visualized differently from the target values.

## Revendications

1. Appareil respiratoire pour la ventilation d'un patient avec des valeurs prédéfinies d'une concentration en oxygène (FiO2) dans le gaz respiratoire, comprenant un dispositif de commande programmable, une source de gaz respiratoire, une source d'oxygène, une unité de mélange de gaz, un affichage (3) et des éléments de contrôle (2) ainsi qu'au moins une interface (8, 18, 28) et un capteur d'oxygène destiné à déterminer une saturation en oxygène (SpO2) du patient, dans lequel le dispositif de commande programmable est configuré pour mettre en œuvre le procédé suivant :
• spécification d'une valeur cible ou de valeurs seuil pour la SpO2 et
• spécification d'une FiO2 dans le gaz respiratoire et
• spécification d'une période de réglage (60) pour la ventilation avec la Fi02 et
• ventilation avec une concentration en oxygène (FiO2) dans le gaz respiratoire conformément à la spécification pour la durée de la période de réglage (60)
• détermination de valeurs de mesure actuelles pour la SpO2 pendant la durée de la période de réglage (60)
• représentation des valeurs de mesure pour la SpO2 sur l'affichage et
• représentation de la spécification pour la Fi02 sur l'affichage,
dans lequel la qualité de signal (40) du signal SpO2 est déterminée, par exemple à l'aide de l'intensité lumineuse, et visualisée de telle façon sur l'affichage (3) qu'une bonne qualité de signal (40) est visuellement représentée autrement qu'une mauvaise qualité de signal,
**caractérisé en ce que** le procédé contient le fait que la ventilation est réalisée avec une concentration en oxygène (FiO2) dans le gaz respiratoire conformément à la spécification pour la durée de la période de réglage (60) et des valeurs de mesure de la SpO2 sont ainsi enregistrées pendant la ventilation et il est analysé si les valeurs de mesure de la SpO2 correspondent à la valeur cible et en cas de déviation définie des valeurs de mesure de la SpO2 par rapport à la valeur cible, une nouvelle valeur prédéfinie est calculée et/ou appliquée pour la FiO2 pour la période de réglage (60) suivante et dans lequel, sur la base de la déviation des valeurs de mesure pour la SpO2 par rapport à la valeur cible, une modification est calculée pour la Fi02 et un symbole (52) est représenté sur l' affichage, dans lequel le symbole (52) fournit à l'observateur une information sur le fait que la valeur prédéfinie suivante pour la FiO2 - en comparaison avec la valeur FiO2 actuelle - demeure inchangée ou est modifiée.

2. Appareil respiratoire selon l'une au moins des revendications précédentes, **caractérisé en ce que** le procédé contient le fait que la durée de la période de réglage (60) est adaptée en fonction de la déviation des valeurs de mesure de la SpO2 par rapport à la valeur cible pour la période de réglage (60) suivante.

3. Appareil respiratoire selon l'une au moins des revendications précédentes, **caractérisé en ce que** le procédé contient le fait que la période de réglage peut être prédéfinie ou est adaptée dans la plage comprise entre 20 et 240 secondes.

4. Appareil respiratoire selon l'une au moins des revendications précédentes, **caractérisé en ce que** le procédé contient le fait qu'un graphe temporel est représenté dynamiquement sur l'affichage pour la période de réglage (60).

5. Appareil respiratoire selon la revendication 1 ou 2, **caractérisé en ce que** le procédé contient le fait que des valeurs de mesure pour la SpO2 et/ou des valeurs prédéfinies pour la FiO2 sont représentées chronologiquement sur l' affichage sous forme de progression.

6. Appareil respiratoire selon l'une au moins des revendications précédentes, **caractérisé en ce que** le procédé contient le fait que des valeurs prédéfinies pour la Fi02 et/ou des valeurs de mesure pour la SpO2 sont représentées sous forme de courbes de progression.

7. Appareil respiratoire selon l'une au moins des revendications précédentes, **caractérisé en ce que** le procédé contient le fait que les courbes de progression pour la SpO2 et la Fi02 sont représentées l'une en dessous de l'autre.

8. Appareil respiratoire selon l'une au moins des revendications précédentes, **caractérisé en ce que** le procédé contient le fait que la valeur de mesure actuelle (31) pour la SpO2 est représentée à la fin de la courbe de progression pour la SpO2 et/ou **en ce que** la valeur prédéfinie actuelle pour la FiO2 est représentée à la fin de la courbe de progression pour la FiO2.

9. Appareil respiratoire selon l'une au moins des revendications précédentes, **caractérisé en ce que** le procédé contient le fait la valeur prédéfinie actuelle pour la FiO2 et/ou la valeur de mesure (31) actuelle pour la SpO2 sont consignées par un symbole (52) à la fin de la courbe de progression, dans lequel l'orientation du symbole (52) est adaptée dynamiquement à l'aide des valeurs de mesure (31) passées.

10. Appareil respiratoire selon l'une au moins des revendications précédentes, **caractérisé en ce que** le procédé contient le fait que la valeur de mesure actuelle pour la SpO2 est représentée sur l'affichage ensemble avec un symbole (52), dans lequel **l'orientation** du symbole (52) est ainsi adaptée dynamiquement de telle façon que l'orientation du symbole fournit à l'observateur une information sur le fait que la valeur prédéfinie suivante pour la FiO2 demeure inchangée ou est modifiée.

11. Appareil respiratoire selon l'une au moins des revendications précédentes, **caractérisé en ce que** le procédé contient le fait que lors d'une baisse définie de la SpO2, un raccourcissement de la période de réglage (60) actuelle et/ou une augmentation de la Fi02 pour la période de réglage (60) suivante sont réalisés.

12. Appareil respiratoire selon l'une au moins des revendications précédentes, **caractérisé en ce que** le procédé contient le fait que pour la SpO2, une valeur cible est visualisée et des valeurs seuil sont visualisées, dans lequel les valeurs seuil sont surlignées en couleur ou visualisées autrement que les valeurs cible.
